# DEMANDE DE BREVET EUROPEEN

(11) **EP 1 477 139 A2**
(43) Date de publication de la demande: **17.11.2004**
(21) Numéro de dépôt: 04291050.5
(22) Date de dépôt: 22.04.2004
(51) Int. Cl.: A61F 2/16

(54) **Systeme pour rouler et comprimer un implant intraoculaire puis l'injecter dans un oeil**

(30) Priorité: 16.05.2003 FR 0305869
(71) Demandeur: CSB PRO, 93600 Aulnay Sous Bois (FR)
(72) Inventeur: Carmona, Francois, 93150 Blanc Mesnil (FR)
(74) Mandataire: Keib, Gérard

(57) **Abrégé**

Il comprend un injecteur 1 avec un piston et des moyens pour rouler et comprimer ledit implant dans l'injecteur, latéralement par rapport à l'axe dudit piston, lesdits moyens comprenant :
- une chambre latérale 10 ménagée dans l'injecteur 1 dans laquelle est posé ledit implant ; et
- un poussoir 3 adapté à coopérer avec ladite chambre latéralement depuis son ouverture extérieure de manière à pousser ledit implant posé dans ladite chambre dans ledit canon de piston.

Le système est caractérisé en ce que la chambre 10 a une hauteur correspondant au diamètre du canon 5 du piston, débouchant perpendiculairement et symétriquement par rapport à l'axe dudit canon, l'extrémité du poussoir présentant une surface hémisphérique de même rayon de courbure que la partie hémisphérique en regard du canon du piston.

## Description

La présente invention concerne un système pour rouler et comprimer un implant intraoculaire, puis l'injecter dans un oeil.

Plus précisément, la présente invention a pour application le domaine des implants intraoculaire, du type notamment commercialisés par la Société Thinoptx^{(R)} sous le nom « Rollable™ ThinLens ». Ces implants sont réalisés en matière synthétique flexible (en général, Acrylique Hydrophile ou silicone) et ultraminces, adaptés à être roulés et comprimés car ils sont destinés à être introduits, en remplacement du cristallin après une phacoémulsification, par une incision que l'on souhaite la plus petite possible pour limiter les problèmes d'astigmatisme, actuellement de l'ordre de 1,5 mm.

Plusieurs solutions ont été proposées pour rouler et comprimer ce type d'implants afin qu'il puisse être injecté par une incision très petite.

La Société Geuder^{(R)} commercialise un système constitué de trois éléments :
- une pince qui sert à saisir un bord longitudinal de l'implant sur la moitié de celui-ci ;
- un insert avec une goulotte dans laquelle l'implant est roulé autour de la pince et un canon cylindrique dans le prolongement de la goulotte dans lequel l'implant une fois roulé autour de la pince est poussé, puis la pince retirée ; et
- un injecteur avec un piston, ledit insert étant placé dans la tête de l'injecteur et orienté de sorte que le piston puisse traverser la goulotte puis pousse l'implant hors du canon cylindrique et de l'injecteur pour l'injecter dans l'oeil.

Les inconvénients majeurs de ce système résident dans :
- l'impossibilité de contrôler le maintien de l'implant enroulé pendant son injection dans l'oeil ;
- le coût élevé lié au nombre d'éléments, à leur complexité, ainsi qu'au temps et au coût nécessaire pour les nettoyer, les décontaminer et les stériliser ; et
- l'impossibilité de guider l'implant dans l'incision.

La Société Duckworth & Kent commercialise un système différent constitué de trois éléments également :
- une plaque de support et de compression comprenant deux niveaux : un premier niveau plan sur lequel est destiné à être posé l'implant, un épaulement hemi-cylindrique et un second niveau plan ;
- un injecteur avec un piston ; l'injecteur est traversé par une fente, s'étendant perpendiculairement à l'axe du cylindre du piston, dans laquelle est destinée à être introduite la plaque de support sur laquelle est placé l'implant ; de manière complémentaire à la plaque, la fente a du côté de l'introduction de la plaque la hauteur du second niveau de celle-ci et un fond hémicylindrique correspondant à la paroi du canon du piston et au-delà la hauteur du premier niveau de la plaque ; ainsi, lorsque la plaque est insérée dans la fente le premier niveau peut traverser l'injecteur tandis que le bord de l'implant rencontre théoriquement le fond hémicylindrique de la fente et se retrouve poussé par l'épaulement pour y être roulé et comprimé ; en bout de course l'épaulement hémicylindrique vient en correspondance avec l'épaulement hémicylindrique de la fente pour recréer un tronçon du canon du piston dans lequel se trouve roulé et comprimé l'implant ;
- un bloc de maintien, sur lequel on pose la seringue et la plaque de support en bout de course, muni de moyens :
- permettant la compression de l'implant avant injection ; et
- empêchant le déplacement latéral de la plaque et donc permettant d'immobiliser l'implant avant l'injection.

Ce système présente pour les mêmes raisons que celles évoquées plus haut l'inconvénient d'être coûteux, difficile à nettoyer, décontaminer et stériliser. Par ailleurs, les deux bords de l'implant sont exposés au pincement de la plaque supportant l'implant coulissant dans l'injecteur et l'implant risque donc de se coincer et d'être abîmé au moment où il est injecté. De plus si l'implant n'est pas injecté immédiatement après la séparation de l'injecteur et du bloc de maintien, l'implant reprend son expansion en se dilatant et ne peut plus être injecté car il reste coincé au niveau de la chambre de compression.

Les deux inconvénients majeurs que l'on retrouve donc dans ces deux systèmes commercialisés sont leur coût trop élevé et le fait que si l'implant n'est pas injecté immédiatement après être enroulé, celui-ci reprend par expansion naturelle sa forme initiale et ne peut plus être injecté.

On connaît également par le brevet US 5,643,275 un système d'injection comprenant deux éléments :
- un injecteur avec un piston ; une chambre latérale, en forme de fente, est ménagée dans le corps de l'injecteur débouchant d'une part dans le canon du piston tangentiellement à une surface arbitrairement inférieure et d'autre part sur l'extérieur de l'injecteur ; l'implant est destiné à être inséré dans cette chambre ; et
- un poussoir destiné à s'introduire dans la chambre, s'ajustant à la dimension de celle-ci, comprenant une extrémité avant concave et une partie arrière pour le manipuler ; en bout de course, l'extrémité avant du poussoir arrive tangentiellement à ladite surface inférieure du canon du piston de l'injecteur de manière à refermer le canon du piston ; le bord avant de l'implant au fond de la chambre est ainsi poussé par le poussoir dans le canon du piston tangentiellement à cette surface inférieure, puis remonte vers le haut autour de la paroi du canon du piston, grâce à laquelle il se retrouve théoriquement roulé et comprimé sur lui-même en spirale lorsque le poussoir est en bout de course.

Ce système présente en pratique les inconvénients suivants :
- comme l'implant est poussé tangentiellement latéralement dans le canon du piston, il est comprimé de manière unilatérale ; il risque donc d'être déformé ;
- à mesure que l'implant s'enroule sur lui-même, il est freiné dans la chambre et se retrouve déformé ;
- on constate en pratique que l'enroulement sur lui-même de l'implant en spirale dépend du l'hydrophilie de l'implant et que les manipulations sont difficilement reproductibles ;
- les variations d'épaisseurs des implants liés à leurs dioptries obligent a concevoir une chambre avec une épaisseur maximale, correspondant à l'épaisseur maximale de l'implant de plus forte dioptrie dans la gamme concernée ; le poussoir plus large risque d'écraser systématiquement les implants de faible dioptries et d'empêcher une reproductibilité dans les manipulations.

La présente invention a pour but notamment de pallier ces inconvénients et de proposer un système qui soit moins coûteux, fiable quelque soit le degré d'hydrophilie et la dioptrie de l'implant, qui n'abîme pas l'implant, qui soit simple à utiliser sans risque d'expansion incontrôlée de l'implant une fois roulé, qui permette une action reproductible, qui puisse être fabriqué par moulage et puisse être à usage unique.

La présente invention propose un système pour rouler et comprimer un implant intraoculaire puis l'injecter dans un oeil, ledit système comprenant un injecteur avec un piston et des moyens pour rouler et comprimer ledit implant dans l'injecteur, latéralement par rapport à l'axe dudit piston, lesdits moyens pour rouler ledit implant comprenant :
- une chambre en forme de fente ménagée dans l'injecteur, ladite chambre étant ouverte d'une part sur le canon du piston et, d'autre part, sur l'extérieur latéralement de l'injecteur, ledit implant étant destiné à être introduit et posé dans ladite chambre depuis l'ouverture extérieure ;
- un poussoir adapté à coopérer avec ladite chambre latéralement depuis son ouverture extérieure de manière à pousser ledit implant posé dans ladite chambre dans ledit canon de piston, ledit poussoir comportant une extrémité avec une surface concave de forme complémentaire à l'ouverture de la chambre sur ledit canon de piston et adaptée à fermer en bout de course le canon du piston,
caractérisé en ce que la chambre pour l'implant a une hauteur correspondant au diamètre du canon du piston, débouchant perpendiculairement et symétriquement par rapport à l'axe dudit canon, l'extrémité du poussoir présentant une surface hémisphérique de même rayon de courbure que la partie hémisphérique en regard du canon du piston.

L'implant est ainsi dans un premier temps poussé en contact sur les deux partie hémisphériques, celle du poussoir et celle formée par la paroi en regard dans le fond du canon du piston, puis roulé grâce à l'action simultané du poussoir agissant sur le bord arrière de l'implant et de ladite paroi hémisphérique du canon de l'injecteur. Puis, dans un deuxième temps, l'action du poussoir va comprimer bilatéralement et sensiblement symétriquement l'implant préalablement roulé autour de la partie hémisphérique du canon de l'injecteur.

Ceci a pour effet de comprimer l'implant de manière plus homogène, d'éliminer les risques de pincement de l'implant et garantir un meilleur glissement de l'implant dans le canon du piston.

L'injecteur et le poussoir, de part leur conception simple, peuvent être réalisés par moulage en matière synthétique, pouvant être choisie pour ses propriétés glissantes facilitant l'enroulement de l'implant, pour son faible taux d'usure, ou pour son faible coût permettant de réaliser un système à usage unique garant de la stérilité.

Selon une caractéristique secondaire préférée, le poussoir est conçu pour que son extrémité hémisphérique puisse être poussé légèrement dans le canon du piston de manière à « sur-comprimer » l'implant dans le tronçon du canon du piston et le bord de l'extrémité hémisphérique du poussoir destiné à être du côté du piston de l'injecteur est chanfreiné de sorte que le piston en s'engageant sur le chanfrein du poussoir relève légèrement celui-ci, alignant le diamètre sous le poussoir dans ledit tronçon avec celui-ci du canon ; le diamètre de l'implant, sensiblement comprimé à un diamètre inférieur du diamètre interne du canon d'injection, n'aura pas le temps de se décomprimer et cela permettra un glissement sans résistance de l'implant durant sa translation dans le canon du piston.

Selon une autre caractéristique secondaire, le système comprend des moyens pour verrouiller le poussoir dans l'injecteur dans une position en bout de course, de manière à immobiliser ledit implant roulé dans le canon du piston, prêt à être poussé par ledit piston hors de l'injecteur dans l'oeil du patient. Cette caractéristique peut être combinée à celle mentionnée ci-dessus, en prévoyant un jeu en translation au niveau des moyens de verrouillage correspondant au léger retrait du poussoir par le piston grâce au chanfrein.

La présente invention sera mieux comprise et d'autres avantages apparaîtront à la lumière de la description qui va suivre d'un exemple de réalisation, description faite en référence aux dessins sur lesquels :
- la figure 1 est une vue de dessus en coupe longitudinale illustrant schématiquement les différentes éléments du système ainsi qu'un implant intraoculaire auquel est destiné ce système ;
- les figures 2 à 4 sont des coupes transversales selon la ligne II-II de la figure 1, illustrant notamment le fonctionnement du système ;
- la figure 5 est une vue partielle en coupe similaire à la figure 2 de l'injecteur et du poussoir illustrant la « sur-compression » possible de l'implant par le poussoir ; et
- la figure 6 est une vue partielle de dessus en coupe du système illustrant également ladite « sur-compression ».

Selon l'exemple de réalisation choisi et représenté sur les figures, le système comprend un injecteur 1 muni d'un piston d'injection 2 et un poussoir 3 servant à pousser, puis rouler et comprimer un implant 4 latéralement dans l'injecteur 1, perpendiculairement à l'axe du canon 5 dudit piston. L'injecteur 1 comprend à proximité de son extrémité d'injection 6 une partie parallélépipédique 7 dont la hauteur correspond au diamètre de l'injecteur et qui se projette latéralement sur un côté avant en une extension avant 8 et sur un côté arrière en une extension arrière 9 (avant et arrière étant choisis ici arbitrairement pour faciliter la description).

Selon l'invention, une chambre 10 est ménagée dans l'extension arrière 9, en forme de fente ayant une hauteur correspondant au diamètre du canon 5 de l'injecteur et débouchant radialement dans celui-ci, tangentiellement aux parois inférieure 11 et supérieure 12. La chambre 10 est destinée à recevoir l'implant 4, posé sur l'une de ses faces inférieure ou supérieure. L'extrémité d'injection 6, orientable avantageusement grâce aux parties d'extension arrière 7 et avant 8, permet un positionnement automatique en regard ou dans l'incision afin de favoriser l'introduction de l'implant dans l'organisme.

Le poussoir 3 est constitué d'une portion poussoir proprement dite 14 destinée à s'engager dans la chambre 10 pour pousser l'implant 4 et qui a ici une forme complémentaire de celle de la chambre 10, notamment en longueur, en largeur et comportant une extrémité concave hémisphérique 15 identique et complémentaire de la paroi hémisphérique 16 en regard du canon 5, présentant un rayon de courbure identique. A son extrémité opposée à l'extrémité hémisphérique 15, le poussoir 3 comporte une portion 18 de dimensions plus grandes, permettant la manipulation du poussoir.

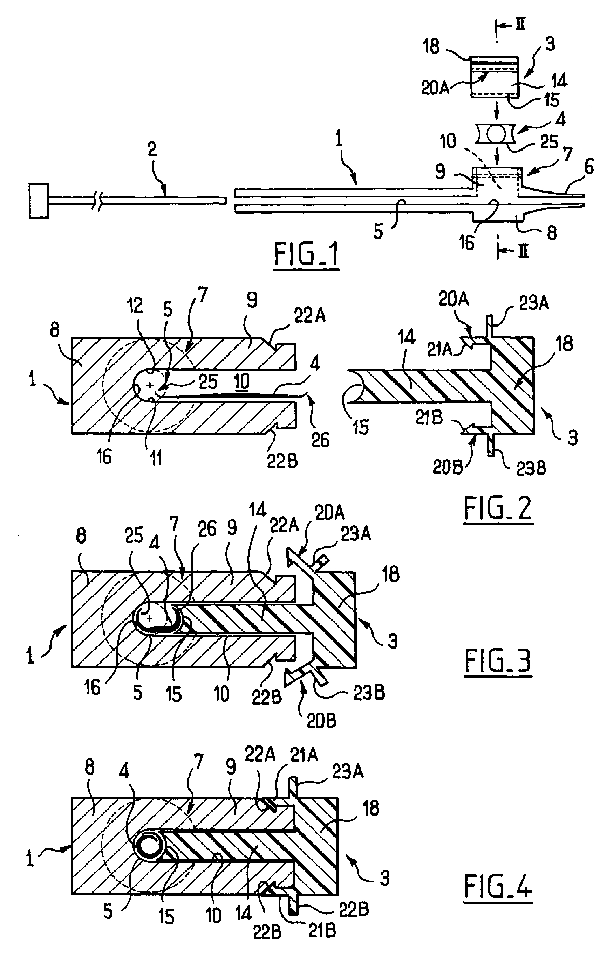

Selon ce mode de réalisation, sont également ménagés des moyens de verrouillage du poussoir en bout de course dans la chambre 10. Ces moyens sont ici constitués de deux projections 20A et 20B, ménagées en direction de l'injecteur lorsque l'on place le poussoir en position de fonctionnement, symétriquement au dessus et en dessous de la portion poussoir 14. Ces projections 20A, 20B, sont munies à leur bord terminal de moyens d'encliquetage 21A, 21B adaptés à coopérer, lorsque la portion poussoir 14 est en bout de course, avec des logements complémentaires 22A, 22B, prévus à cet effet sur l'extension arrière 9 de l'injecteur. Des pattes 23A, 23B, se projetant perpendiculairement sur l'extérieur, sont également prévues au niveau de la zone d'attache des projections 20A, 20B à la portion 18, servant à libérer les moyens d'encliquetage 21A et 21B, ceci grâce à une certaine flexibilité du matériau choisi (comme cela est schématisé à la figure 3).

Le système fonctionne de la manière suivante. L'implant 4 a en général une forme rectangulaire. Il est introduit latéralement dans la chambre 10 selon l'un de ses bords longitudinaux, référencé 25 ici, comme on le voit à la figure 1, de préférence légèrement enfoncé à l'intérieur de la chambre 10. Le poussoir 3 est placé en regard de l'ouverture sur l'extérieur de la chambre 10, avec les moyens de verrouillage 20A, 20B, en regard des moyens complémentaires 22A et 22B de l'injecteur. A mesure que le poussoir 3 est poussé, le bord 25 de l'implant 4 avance vers le fond de la chambre 10, pénètre tangentiellement dans le canon 5 de l'injecteur au niveau de sa paroi inférieure 11, puis s'enroule le long de la paroi hémisphérique du canon, en remontant la paroi de fond 16 du canon jusqu'à la paroi supérieure 12. Simultanément le bord arrière 26 de l'implant 4 remonte sur la face 15 du poussoir de manière symétrique au bord opposé. La longueur du poussoir (portion 14) et la profondeur de la chambre 10 sont adaptées selon l'invention aux dimensions de l'implant pour que l'extrémité hémisphérique du poussoir sollicite et comprime ainsi les deux bords opposés de l'implant de façon totalement symétrique. L'action ensuite simultanée et sensiblement symétrique du poussoir sur les deux bords longitudinaux 25 et 26 de l'implant va pour finir enrouler de manière homogène l'implant sur lui-même au niveau du canon de l'injecteur jusqu'à la position de fin de course représentée à la figure 4. Selon l'invention, et comme on le voit sur cette figure l'implant 4, aura fortement tendance à s'enrouler en spirale. Comme on le comprend d'après ce fonctionnement détaillé, les risques de pincement de l'implant sont grâce à la forme hémisphérique du poussoir fortement réduits. Les difficultés liées aux variations d'épaisseur d'implant sont évitées et les difficultés liées à l'expansion des implants sont contrôlées.

L'implant 4 comprimé comme sur la figure 4 et bloqué dans cette position grâce aux moyens de verrouillage se trouve en alignement avec le piston 2 et peut être injecté immédiatement dans l'oeil du patient ou ultérieurement.

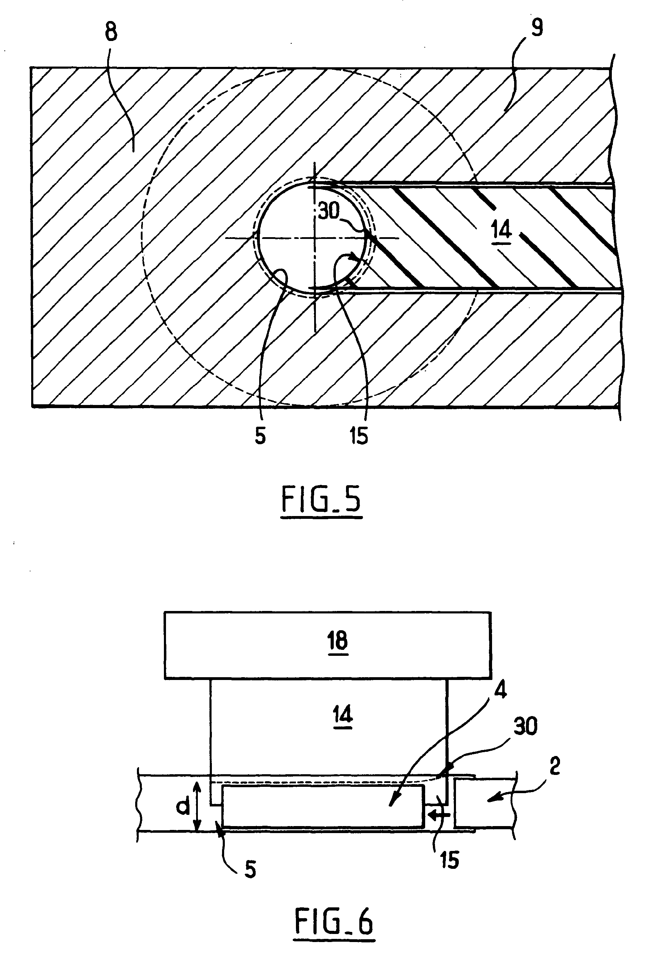

Aux figures 5 et 6, on a représenté une particularité de l'invention permettant, comme les moyens de verrouillage, de faciliter le glissement de l'implant dans le canon du piston, cette particularité pouvant coexister avec des moyens de verrouillage ou pouvant être mise en oeuvre séparément seule.

Les moyens similaires à ceux décrits plus haut en référence aux figures 1 à 4 portent ici les mêmes numéros de référence.

Selon cette particularité de l'invention, le poussoir est dimensionné pour pouvoir avancer dans le canon du piston et surcomprimer l'implant à un diamètre d (voir figure 6) inférieur au diamètre interne dudit canon. En outre, l'extrémité hémisphérique 15 du poussoir 14 comporte du côté du piston 2 un chanfrein 30 qui est tel que lorsque le piston 2 est poussé au contact du poussoir, il provoque avantageusement le retrait du poussoir pour aligner le diamètre au niveau de la chambre de compression où se trouve l'implant avec celui-ci du canon du piston et permettre le passage du piston.

En cas d'arrêt de la poussée sur le poussoir, contrairement aux dispositifs de l'art antérieur, il n'y a pas de blocage. Par simple pression du poussoir, une compression est possible à tout moment. Ainsi, l'implant n'a pas besoin d'être injecté immédiatement.

Il va de soi que des variantes de réalisation sont possibles notamment en ce qui concerne la forme de l'injecteur, de l'extension dans laquelle est ménagée la chambre 10, et des moyens de verrouillage du poussoir.

## Revendications

1. Système pour rouler et comprimer un implant intraoculaire (4) puis l'injecter dans un oeil, ledit système comprenant un injecteur (1) avec un piston (2) et des moyens (3,10) pour rouler et comprimer ledit implant dans l'injecteur, latéralement par rapport à l'axe dudit piston, lesdits moyens pour rouler ledit implant comprenant :
- une chambre (10) en forme de fente ménagée dans l'injecteur (1), ladite chambre étant ouverte d'une part sur le canon (5) du piston (2) et, d'autre part, sur l'extérieur latéralement de l'injecteur, ledit implant étant destiné à être introduit et posé dans ladite chambre depuis l'ouverture extérieure ;
- un poussoir (3) adapté à coopérer avec ladite chambre latéralement depuis son ouverture extérieure de manière à pousser ledit implant posé dans ladite chambre dans ledit canon de piston, ledit poussoir comportant une extrémité (15) avec une surface concave de forme complémentaire à l'ouverture de la chambre sur ledit canon de piston et adaptée à fermer en bout de course le canon du piston,
**caractérisé en ce que** la chambre (10) pour l'implant a une hauteur correspondant au diamètre du canon (5) du piston, débouchant perpendiculairement et symétriquement par rapport à l'axe dudit canon, l'extrémité du poussoir présentant une surface hémisphérique de même rayon de courbure que la partie hémisphérique en regard du canon du piston.

2. Système selon la revendication 1, **caractérisé en ce que** le poussoir est adapté à surcomprimer l'implant à un diamètre inférieur à celui du canon du piston, un chanfrein (30) étant ménagé sur le côté de la surface hémisphérique (15) de l'extrémité du poussoir destiné à coopérer avec le piston de sorte que ce dernier puisse provoquer le retrait du poussoir dans ladite chambre et permettre son passage.

3. Système selon la revendication 1 ou 2, **caractérisé en ce qu'**il comprend des moyens verrouillage (20A,20B) du poussoir (3) dans l'injecteur en bout de course, de manière à immobiliser ledit implant roulé dans ledit canon du piston, prêt à être poussé par ledit piston hors de l'injecteur dans l'oeil du patient.

4. Système selon la revendication 2 et 3, **caractérisé en ce que** les moyens de verrouillage présentent un jeu en translation permettant l'avancée légèrement du poussoir par rapport au diamètre du canon du piston de manière à sur-comprimer l'implant et le retrait au passage du piston.

5. Système selon la revendication 3 ou 4, **caractérisé en ce que** les moyens de verrouillage (20A, 20B) comprennent des moyens d'encliquetage (21A,21 B) ménagés dans le poussoir (3) adaptés à coopérer en bout de course du poussoir avec des logements complémentaires (22A, 22B) ménagés dans l'injecteur (1), et des moyens (23A, 23B) permettant de libérer les moyens de verrouillage (20A, 20B) desdits logements (22A, 22B).

6. Système selon l'une des revendications précédentes, **caractérisé en ce que** l'injecteur (1) est réalisé par moulage en matériau synthétique.

7. Système selon l'une des revendications précédentes, **caractérisé en ce que** le poussoir (3) est réalisé par moulage en matériau synthétique.

8. Système selon les revendications 5 et 7, **caractérisé en ce que** les moyens de verrouillage sont constitués de projections (20A,20B) en regard de l'extrémité hémisphérique du poussoir (3) dont le bord terminal est muni desdits moyens d'encliquetage (21A,21B), les moyens pour libérer lesdits moyens d'encliquetage étant constitués de pattes permettant de faire fléchir lesdites projections pour les écarter de leurs logements (22A,22B) d'encliquetage.

9. Système selon l'une quelconque des revendications précédentes, **caractérisé en ce que** la longueur du poussoir (3) et la profondeur de la chambre (10) sont dimensionnées de manière que l'implant puisse être dans un premier temps enroulé dans le canon du piston et dans ladite chambre (10), puis dans un deuxième temps comprimé bilatéralement dans le canon du piston.

10. Système selon l'une des revendications précédentes, **caractérisé en ce que** l'injecteur (1) comprend à proximité de son extrémité d'injection (6) une partie (7) qui se projette latéralement sur un côté arrière en une extension arrière (9) dans laquelle est ménagée ladite chambre (10).

11. Système selon la revendication 10, **caractérisé en ce que** ladite partie (7) se projette latéralement sur un côté avant en une extension avant (8) permettant un positionnement en regard de l'extrémité d'injection (6) ou dans l'incision afin de favoriser l'introduction de l'implant dans l'organisme.

12. Système selon la revendication 10 ou 11, **caractérisé en ce que** ladite partie (7) se projetant latéralement est parallélépipédique.
